# EUROPÄISCHE PATENTSCHRIFT

(11) **EP 2 699 210 B1**
(45) Veröffentlichungstag und Bekanntmachung des Hinweises auf die Patenterteilung: **18.02.2015**
(21) Anmeldenummer: 12712650.6
(22) Anmeldetag: 30.03.2012
(51) Int. Cl.: A61F 13/15, A61F 13/505

(54) **FIXIER- UND TRAGEVORRICHTUNG FÜR EINE WEGWERFBARE ABSORBIERENDE INKONTINENZVORLAGE**
FASTENING AND CARRYING DEVICE FOR A DISPOSABLE ABSORBENT INCONTINENCE PAD
DISPOSITIF DE FIXATION ET DE SUPPORT POUR UNE PROTECTION ABSORBANTE JETABLE POUR INCONTINENCE

(30) Priorität: 20.04.2011 DE 102011007820
(43) Veröffentlichungstag der Anmeldung: 26.02.2014
(73) Patentinhaber: Paul Hartmann AG, 89522 Heidenheim (DE)
(72) Erfinder: PAZ, Rui Miguel, 47800 Krefeld (DE); KESSELMEIER, Ruediger, 89542 Herbrechtingen (DE); MALOWANIEC, D. Krzysztof, 89522 Heidenheim (DE); DRUMEVA-EBERIUS, Albena, 89522 Heidenheim (DE); NAGY, Uwe, Heidenheim 89522 (DE); SWEREV, Maximilian, 86169 Augsburg (DE)
(74) Vertreter: Dreiss, Uwe
(86) Internationale Anmeldenummer: PCT/EP2012/055876
(87) Internationale Veröffentlichungsnummer: WO 2012/143229

(56) Entgegenhaltungen:
- WO-A1-2010/106926
- US-A- 4 022 212
- US-A1- 2006 247 599

## Beschreibung

Die Erfindung betrifft eine Fixier- und Tragevorrichtung für eine wegwerfbare absorbierende Inkontinenzvorlage mit einem mittels Gürtelverschlusselementen auf sich selbst lösbar verschließbaren und so in Hüftumfangsrichtung durchgehende Hüftöffnung bildenden Hüftgürtel, an dem die Inkontinenzvorlage lösbar fixierbar ist, so dass sie im Schrittbereich des Benutzers getragen und nach Gebrauch wieder vom Hüftgürtel entfernt werden und verworfen werden kann, wobei der Hüftgürtel einen vorderen Bauchbereich, einen hinteren Rückenbereich und einen linken und einen rechten Seitenbereich umfasst und der Hüftgürtel ausgehend von dem Rückenbereich und dem Bauchbereich je einen sich in einer Längsrichtung in Richtung auf den Schrittbereich des Benutzers erstreckenden Latzabschnitt aufweist, der an seiner körperzugewandten Seite Verschlusselemente aufweist, die mit komplementären Verschlusselementen auf der körperabgewandten Seite der Inkontinenzvorlage lösbar haftend zusammenwirken und wobei die Verschlusselemente des Latzabschnitts vor der Ingebrauchnahme auf die körperzugewandte Seite des Latzabschnitts eingeschlagen und in dieser Konfiguration leicht lösbar an der körperzugewandten Seite des Latzabschnitts festgelegt sind.

Eine derartige Fixier- und Tragevorrichtung ist beispielsweise aus der WO 2004/069122 A1 bekannt. Bei dieser bekannten Fixier- und Tragevorrichtung wird der Hüftgürtel im Bauchbereich auf sich selbst geschlossen. Im Seitenbereich links und rechts zwischen Bauchbereich und Rückenbereich erstreckt sich der Hüftgürtel nach unten und bildet dort jeweils einen latzartigen Ansatz, an den eine verhältnismäßig breit ausladende absorbierende Windeleinheit festgelegt werden kann, wobei auf der körperzugewandten Seite der absorbierenden Windeleinheit beidseits vorn und hinten Verschlusselemente hierfür vorgesehen sind.

Aus der EP 0 700 278 B1 ist eine sogenannte Gürtelwindel mit einem auf sich selbst schließbaren verhältnismäßig schmalen Hüftgürtel bekannt, an dessen körperabgewandter Seite eine nach Art einer Windel anmutende absorbierende Einheit festlegbar ist.

US 2006/247599 A1 offenbart eine Fixer- und Tragevorrichtung für eine wegwerfbare absorbierende Inkontinenzvorlage mit einem mittels Gürtelverschlusselementen auf sich selbst lösbar schließbaren Hüftgürtel, an den die Inkontinenzvorlage lösbar anbringbar ist.

Schließlich zeigt die DE 10 2009 049 463 eine gattungsgemäße Gestaltung, aus der es bereits bekannt ist, die Verschlusselemente, die an den Latzabschnitten vorgesehen sind, und die sich im Rückenbereich und im Bauchbereich in den Schrittbereich des Benutzers erstrecken, auf sich selber umzuschlagen und so Verschlusselemente, die im Latzabschnitt angeordnet sind, festlegen zu können.

Hierdurch wird bereits eine einfachere Anlegbarkeit der Fixier- und Tragehilfe erreicht.

Aufgabe der Erfindung ist es nun, die Anlegbarkeit zum einen weiter zu verbessern und zum anderen eine gattungsgemäße Fixier- und Tragevorrichtung dahingehend weiterzubilden, dass vor Ingebrauchnahme ein Kontakt der Verschlusselemente mit dem Träger vermieden wird und auch in der Form, in der die Fixier- und Tragevorrichtung vor in Ingebrauchnahme bereitgestellt wird, ein Verhaken von Verschlusselementen, die an den Latzabschnitten festgelegt sind, sowie von Gürtelverschlusselementen, sicher verhindert werden kann, so dass eine einfache Entnahme einzelner Produkte aus einer Verpackung oder von einem Stapel mehrerer Produkte gewährleistet werden kann.

Diese Aufgabe wird gelöst durch eine Fixier- oder Tragevorrichtung der gattungsgemäßen Art, bei der die Verschlusselemente des Latzabschnitts vor der Ingebrauchnahme auf die körperzugewandte Seite des Latzabschnitts eingeschlagen sind, und wobei der Latzabschnitt mit den darauf eingeschlagenen Verschlusselementen auf den vorderen Bauch- und hinteren Rückenbereich eingeschlagen ist. Dabei werden die Verschlusselemente mit komplementären Elementen des Latzabschnitts in Zusammenwirkung gebracht, und zwar unter Verwendung von vorzugsweise mechanisch und/oder klebend wirkenden Verschlusselementen.

Durch den in Richtung auf den Schrittbereich des Benutzers erstreckten Latzabschnitt bildet der Hüftgürtel zugleich die grundsätzliche Konfiguration eines Höschens, welches zur Vollendung der Höschenform nur noch durch einen Schrittbereich ergänzt zu werden braucht. Dieser Schrittbereich wird bei der erfindungsgemäßen Fixier- und Tragevorrichtung durch die absorbierende Inkontinenzvorlage gebildet, wobei der hintere und vordere Latzabschnitt mit der körperabgewandten Seite der Inkontinenzvorlage lösbar haftend zusammenwirken, und zwar unter Verwendung von vorzugsweise mechanischen und/oder klebenden Verschlusselementen, nachdem die Fixier- oder Tragevorrichtung an einem Träger angelegt worden ist. Durch die Fixierung bzw. das Einschlagen der Verschlusselemente auf den Latzabschnitt sowie das Einschlagen des Latzabschnitts auf den vorderen Bauch- bzw. hinteren Rückenbereich wird erreicht, dass die insbesondere mechanisch und/oder klebend wirkenden Verschlusselemente während des Anlegens des Hüftgürtels insbesondere bei pflegebedürftigen Personen deaktiviert sind. Sie behindern dabei nicht das Anlegen des Hüftgürtels, indem sie am Hüftgürtel oder an anderen Komponenten, wie Bettlaken, Patientenunterlagen oder dergleichen, festhaken. Die Faltung wird dann erst nach Anlegen des Hüftgürtels geöffnet, um die Vorlage zu fixieren.

Besonders vorteilhaft ist dabei, wenn das zweimalige Einschlagen, nämlich der Verschlusselemente auf den Latzabschnitt und der Latzabschnitte auf den vorderen bzw. hinteren Bauchbereich derart erfolgt, dass im entsprechend vorliegenden Zustand der Hüftgürtel annähernd über seine gesamte Länge die gleiche Erstreckung in Längsrichtung des Trägers aufweist.

Bevorzugt ist eine Fixier- und Tragevorrichtung mit auf den Bauch- und Rückenbereich eingeschlagenen Verschlusselementen des Latzabschnitts im Bauch- und Rückenbereich, die lediglich die Erstreckung in Längsrichtung des vorderen Bauchbereichs oder hinteren Rückenbereichs aufweist.

Weiterhin bevorzugt ist eine Fixier- und Tragevorrichtung der zuvor beschriebenen Konfiguration, bei der die Erstreckung des linken und rechten Seitenbereichs in Längsrichtung der Erstreckung des vorderen Bauchbereichs und/oder des hinteren Rückenbereichs in Längsrichtung entspricht.

Der Hüftgürtel umfasst in Hüftumfangsrichtung einen Bauchbereich sowie einen Rückenbereich und zwei Seitenbereiche, die jeweils eine Erstreckung in Hüftumfangsrichtung aufweisen und sich in Hüftumfangsrichtung aneinander anschließen bei Bildung eines geschlossenen Hüftgürtels.

In Längsrichtung besitzen Bauch- und Rückenbereich sowie die Seitenbereiche eine proximale und eine distale Querkante, die gerade oder gekrümmt, insbesondere auch nur bereichsweise gekrümmt ausgebildet sein können, zur Ausbildung einer Konturierung.

Schließlich wird die distale Erstreckung des Bauch- und Rückenbereichs und damit die distale Querkante des Bauch- und Rückenbereichs definiert durch eine gedachte Linie in Querrichtung am proximalsten Punkt der distalen Begrenzung des Hüftgürtels, beispielsweise eine Tangente. Der proximalste Punkt kann dabei in Hüftumfangsrichtung im Bauch-, Rücken- oder Seitenbereich liegen. Dabei ist die Fläche proximal dieser distalen Querkante ein Teil des Bauch- bzw. Rückenbereichs und die Fläche distal dieser distalen Querkante ein Teil des jeweiligen Latzabschnitts.

Als Längsrichtung des Hüftgürtels wird dabei die Längsrichtung an einem stehenden Benutzer definiert, die Querrichtung ist dabei senkrecht hierzu und fällt mit der Hüftumfangsrichtung zusammen. Die Längsrichtung der Inkontinenzvorlage entspricht der Richtung deren Längserstreckung, wobei die Querrichtung hierzu senkrecht verläuft.

Besonders bevorzugt weist die Inkontinenzvorlage zwei Längs- und zwei Querkanten auf, wobei im an der Fixier- und Tragevorrichtung festgelegten Zustand die Querkanten der Inkontinenzvorlage vorzugsweise im Wesentlichen einer Querkante, insbesondere der dem Schrittbereich abgewandten, also proximalen Querkante des Hüftgürtels entsprechen. Das heißt, die proximale Kante des Bauch- oder Rückenbereichs fällt mit einer Querkante der Inkontinenzvorlage in angelegten Zustand vorzugsweise zusammen. Im Falle einer konturierten proximalen Querkante des Hüftgürtels, beispielsweise durch einen Bauchausschnitt, läuft die proximale Kante parallel zur Hüftumfangsrichtung durch den distalsten Punkt dieser Konturierung. Auf diese Weise kann einfach eine Markierung bereitgestellt werden, anhand derer die optimale Anlegung des Hüftgürtels sowie der Inkontinenzvorlage am Hüftgürtel erreicht werden kann und somit auch für die jeweilige Größe optimale Positionierung der Verschlusselemente.

Zur Verbesserung der Passform und für eine einfache Handhabung erweist es sich als vorteilhaft, wenn der betreffende Latzabschnitt sich in Richtung auf den Schrittbereich verjüngt. Weiter erweist es sich als vorteilhaft, wenn die Verschlusselemente des Latzabschnitts an einem schrittzugewandten Ende des Latzabschnitts vorgesehen sind. Darüber hinaus können am hinteren und/oder vorderen Latzabschnitt ein oder mehrere weitere Verschlusselemente vorgesehen sein, die zwischen dem schrittzugewandten Ende des Latzabschnitts und dem proximalen Rand des Hüftgürtels, inbesondere im Bereich der Latzabschnitte positioniert sind und die der zusätzlichen Fixierung der Inkontinenzvorlage dienen.

Weiter erweist es sich als vorteilhaft, wenn die vorzugsweise mechanisch und/oder klebend wirkenden Verschlusselemente des Latzabschnitts insbesondere am schrittzugewandten Ende des Latzabschnitts auf einem streifenförmigen und quer zur Längsrichtung erstreckten Materialabschnitt vorgesehen sind, der an den Latzabschnitt angefügt ist. Auf diese Weise kann der Latzabschnitt über seine gesamte flächenhafte Erstreckung in Querrichtung in einer bestimmungsgemäßen zum Benutzer symmetrischen Weise an der Außenseite (auch Rückenschicht oder Backsheet) der Inkontinenzvorlage lösbar festgelegt werden. Der Latzabschnitt kann so weitgehend faltenfrei und wie erwähnt zu den Beinen und zum Schrittbereich bzw. zu einer auf den Benutzer abstellenden Längsmittelachse korrekt ausgebreitet und fixiert werden.

Vorzugsweise sind die Verschlusselemente in dieser Position 100 mm bis 180 mm, vorzugsweise 120 mm bis 170 mm, weiter bevorzugt 140 mm bis 160 mm im Bereich des vorderen Latzabschnitts und vorzugsweise 170 mm bis 250 mm, vorzugsweise 190 mm bis 240 mm, weiter bevorzugt 210 mm bis 230 mm im Bereich des hinteren Latzabschnitts von der proximalen Querkante des Hüftgürtels entfernt.

Vorzugsweise haben die mechanisch und/oder klebend wirkenden Verschlusselemente des Latzabschnitts eine Erstreckung in Querrichtung von 8 cm bis 14 cm, vorzugsweise 10 cm bis 12 cm, vorzugsweise 11 cm und vorzugsweise eine Erstreckung in Längsrichtung von 0,5 cm bis 5,0 cm, vorzugsweise 1,0 cm bis 4,0 cm, vorzugsweise 1,0 cm bis 3,0 cm, vorzugsweise 1,5 cm. Damit ist eine ausreichend große Fläche für die Haftverbindung der mechanisch und/oder klebend wirkenden Verschlusselemente des Latzabschnitts mit der Außenseite der Inkontinenzvorlage für einen sicheren Produktsitz gewährleistet.

Die Außenseite der Inkontinenzvorlage ist dabei vorzugsweise so ausgebildet, dass sie mit den Verschlusselementen des Latzabschnittes korrespondiert, das heißt, bei einem mechanischen Verschlusselement in Form von Haken wird die Außenseite der Inkontinenzvorlage vorzugsweise durch eine schlaufenbildende Komponente, vorzugsweise durch ein Vlies, insbesondere die Vlieslage eines Vlies-Folien-Laminates, gebildet, während bei einem klebenden Verschlusselement die Außenseite der Inkontinenzvorlage vorzugsweise durch eine Folienlage gebildet wird, auf der die klebenden Verschlusselemente anhaften können.

Darüber hinaus ist vorzugsweise vorgesehen, dass der Latzabschnitt mit auf sich eingeschlagenem Verschlusselement des Latzabschnitts noch einmal derart um eine quer zur Längsrichtung verlaufende Faltlinie eingeschlagen wird, dass der Latzabschnitt vollständig auf dem Bauch- oder Rückenbereich zu liegen kommt, vorzugsweise auf der Aussenseite des Bauch- oder Rückenbereichs. Diese Faltung des Latzabschnitts in zweifacher Weise, so dass der Latzabschnitt vollständig auf dem Bauch- bzw. Rückenbereich zu liegen kommt, vorzugsweise sowohl für den vorderen als auch für den hinteren Latzabschnitt, besitzt den Vorteil, dass die Faltung erst nach dem Anlegen des Hüftgürtels und der Inkontinenzvorlage geöffnet wird, um die Vorlage zu fixieren, und hierdurch ein leichteres Hindurchfädeln des Hüftgürtels, der dann im Wesentlichen über die gesamte Länge des Hüftgürtels eine einheitliche Breite aufweist, unter einem liegenden Träger erfolgen kann und es insbesondere nicht zu einem Anhaften der Verschlusselemente an weiteren Bekleidungsstücken oder der Haut des Patienten oder Wäschestücken kommen kann.

Zusätzlich kann diese Faltung der Latzabschnitte durch Fixierpunkte festgelegt werden. Dies kann zum Beispiel mittels einer Ultraschallverbindung, insbesondere kleiner separater Schweißpunkte erfolgen, die im Randbereich der überlappenden Lagen befestigt werden, aber auch mittels Klebeverbindungen, insbesondere Klebepunkten. Andere Arten der Anhaftung sind ebenfalls umfasst. Die Öffnungskraft dieser zusätzlichen Fixierung der Latzfaltung liegt signifikant unterhalb der Materialfestigkeit des Hüftgürtels und zerstört beim Auffalten der Latzabschnitte nicht das Material des Hüftgürtels.

Im Hinblick auf die praktische Handhabbarkeit erweist es sich auch als vorteilhaft, wenn der Hüftgürtel nur in einem Bereich öffenbar und schließbar und die Gürtelverschlusselemente in diesem Bereich an freien Endabschnitten des Hüftgürtels vorgesehen sind. Der Hüftgürtel ist also langgestreckt und hat zwei Endabschnitte, die mittels der Gürtelverschlusselemente aufeinander schließbar sind, um den Hüftgürtel um den Körperumfang des Benutzers herum anlegen zu können.

In Weiterbildung dieses Erfindungsgedankens erweist es sich als vorteilhaft, wenn im anderen Seitenbereich, in dem der Gürtel also nicht öffenbar und schließbar ist, Sekundärverschlusselemente vorgesehen sind, mittels derer eine Umfangslänge des Hüftgürtels einstellbar und so zumindest der vordere Latzabschnitt symmetrisch zum Schritt des Benutzers positionierbar ist. Vorzugsweise sind die Sekundärverschlusselemente so ausgebildet, dass sie im angelegten Zustand der Fixier- und Tragevorrichtung in der Ansicht optisch und/oder haptisch nicht von den Gürtelverschlusselementen unterscheidbar sind. Es kann sich jedoch auch als vorteilhaft erweisen, wenn die Sekundärverschlusselemente von den Gürtelverschlusselementen optisch und/oder haptisch unterscheidbar sind, um auf die unterschiedliche Funktion der Verschlusselemente hinzuweisen. Dies kann beispielsweise durch unterschiedliche Farben, Formen, Textur oder auf andere Weise erfolgen. Die Gürtelverschlusselemente und die Sekundärverschlusselemente sind dabei vorteilhafter Weise beidseits des Bauchbereichs und/oder in Seitenbereichen des Hüftgürtels vorgesehen.

Beim Anlegen des Hüftgürtels führt die Pflegeperson den geöffneten langgestreckten Hüftgürtel bei einem liegenden Patienten unter dessen Körper auf Hüft- oder Rückenhöhe hindurch. Sodann wird der Gürtel zur Bildung der geschlossenen Hüftöffnung mittels der Gürtelverschlusselemente auf sich selbst geschlossen. In diesem Zustand ist der Gürtel zwar angelegt, jedoch noch nicht durch Aktivierung der Sekundärverschlusselemente in seine optimale Passform gebracht. Beispielsweise wird in diesem Zustand der bauchseitige Latzabschnitt durch den Zug in Hüftumfangsrichtung, der durch das Schließen der Gürtelverschlusselemente ausgeübt wird, zu der betreffenden Seite hin gezogen. Dem kann nun durch Einstellen der optimalen Hüftumfangslänge und einer geeigneten Spannung in Hüftumfangsrichtung begegnet werden, indem die Sekundärverschlusselemente entsprechend optimal eingestellt werden. Mittels dieser Sekundärverschlusselemente werden also - wie bereits ausgeführt - nicht zwei offene freie Enden aufeinander geschlossen, sondern es wird die Umfangslänge des Hüftgürtels gegebenenfalls verstellt.

Die Sekundärverschlusselemente können in verschiedener Weise verwirklicht sein. Nach einer bevorzugten Ausführungsform der Erfindung wird vorgeschlagen, dass eine Komponente der Sekundärverschlusselemente auf einem an den Hüftgürtel angefügten Materialabschnitt vorgesehen ist und die andere Komponente an einer Außenseite des Hüftgürtels selbst vorgesehen ist. Solchenfalls wird also der Hüftgürtel als solcher nicht verändert, sondern er erhält ein zusätzliches Element in Form des die Sekundärverschlusselemente tragenden Materialabschnitts, der in an sich beliebiger Weise auf das Material des Hüftgürtels aufgebracht werden kann, beispielsweise durch Aufnähen, Aufkleben, Aufsiegeln, Ultraschallschweißen oder dergleichen übliche Fügeverfahren.

Es erweist sich auch als vorteilhaft, wenn sich die Gürtelverschlusselemente und/oder die Sekundärverschlusselemente über im Wesentlichen die gesamte Erstreckung des Hüftgürtels in Längsrichtung, also über die gesamte Breite des Hüftgürtels, erstrecken. Auf diese Weise kann beim Einstellen der Sekundärverschlusselemente ein gleichmäßiger Zug über die gesamte Breite des Gürtels auf den Gürtel ausgeübt werden.

Es ist auch denkbar, dass sich die Gürtelverschlusselemente und/oder die Sekundärverschlusselemente zu ihrem freien Ende hin verjüngen oder verschmälern, um ein Reiben der Ecken dieser Verschlusselemente auf der Haut des Benutzers zu minimieren.

In einer alternativen Ausführungsform ist es darüber hinaus denkbar, dass die Gürtelverschlusselemente und/oder die Sekundärverschlusselemente sich nicht über im Wesentlichen die gesamte Breite des Hüftgürtels erstrecken. In einem solchen Fall ist es besonders vorteilhaft, wenn die Quererstreckung der Gürtelverschlusselemente und/oder der Sekundärverschlusselemente größer ist als ihre Längserstreckung.

Vorzugsweise stehen die Gürtelverschlusselemente und/oder die Sekundärverschlusselemente über den Seitenrand des Hüftgürtels um mindestens 30 mm, insbesondere um mindestens 40 mm und weiter insbesondere um mindestens 50 mm in Querrichtung über. Die Erstreckung der Gürtelverschlusselemente und/oder die Sekundärverschlusselemente in Längsrichtung der Fixier- und Tragevorrichtung beträgt insbesondere mindestens 25 mm, weiter insbesondere mindestens 35 mm und weiter insbesondere mindestens 45 mm. Damit ergibt sich ein bevorzugtes Verhältnis von Quererstreckung zu Längserstreckung von 30 zu 25, vorzugsweise von 40 zu 35 und insbesondere von 50 zu 45. Insbesondere kann vorgesehen sein, dass die Gürtelverschlusselemente und/oder die Sekundärverschlusselemente trapezförmig ausgebildet sind, insbesondere mit einem Seitenverhältnis von 50mm in Querrichtung zu 35/30mm in Längsrichtung, wobei die kürzere Seite des Trapezes die freie Seite der Verschlusselemente bildet. Damit ist eine ausreichend große Fläche für die Haftverbindung der Gürtelverschlusselemente und/öder der Sekundärverschlusselemente mit der Außenseite des Hüftgürtels für einen sicheren Produktsitz gewährleistet.

Dabei können die Gürtelverschlusselemente und/oder die Sekundärverschlusselemente sogenannte Anfass- oder Griffabschnitte umfassen oder mit diesen verbunden sein. Die Anfass- oder Griffabschnitte sind vorzugsweise am freien Ende der Gürtel- oder Sekundärverschlusselemente vorgesehen und weisen keine Verschlussmittel z.B. in Form von mechanischen und/oder klebenden Elementen auf. Hierdurch wird die Anfassbarkeit der Verschlussmittel verbessert.

In Weiterbildung der Erfindung wird vorgeschlagen, die Gürtelverschlusselemente und/oder die Sekundärverschlusselemente zumindest abschnittsweise elastisch auszubilden.

Zum Verkürzen der Umfangslänge des Gürtels unter Verwendung der Sekundärverschlusselemente wird dabei vorzugsweise eine Z-förmige Faltung des Gürtels durch Schließen der Sekundärverschlusselemente bewirkt, die durch den dabei entstehenden Druck des Hüftgürtels gegen die Körperoberfläche des Benutzers und/oder durch Reibung der aneinander anliegenden Abschnitte der Z-förmigen Faltung gehalten wird.

Im Hinblick auf die Schaffung einer guten Passform und eines hohen Tragkomforts erweist es sich als vorteilhaft, wenn der Hüftgürtel mindestens einen elastischen Abschnitt, vorzugsweise in jedem Seitenbereich einen elastischen Abschnitt umfasst, so dass der Hüftgürtel in Hüftumfangsrichtung elastisch dehnbar ist.

Es erweist sich weiter als vorteilhaft, wenn der Hüftgürtel im Bauchbereich und/oder im Rückenbereich im Wesentlichen undehnbar ausgebildet ist.

In einer alternativen Ausführungsform kann es sich jedoch auch als vorteilhaft erweisen, wenn sich ein oder mehrere elastische Abschnitte im Bauchbereich und/oder Rückenbereich befinden, vorzugsweise in Form einer keilförmigen, trapezförmigen oder viereckigen Elastifizierung. Eine besonders bevorzugte alternative Ausführungsform weist zwei trapezförmige Elastifizierungen im Rückenbereich auf, die insbesondere als elastisch dehnbare Materialabschnitte ausgebildet sind, die jeweils in den Bereichen des Rückenbereichs vorgesehen sind, die an die Seitenbereiche angrenzen und zwischen sich einen nicht elastischen Bereich einschließen. Dabei können die beiden parallelen Kanten der elastischen Materialabschnitte in Längsrichtung angeordnet sein und die beiden weiteren Kanten mit einer proximalen Querkante des Hüftgürtels und mit einer auf den Schrittbereich gerichteten Kante des Rückenbereichs zusammenfallen. Je nach Breite der elastischen Bereich kann vorgesehen sein, dass diese sich in Längsrichtung bis in den Latzabschnitt, insbesondere den hinteren Latzabschnitt erstrecken.

Im Hinblick auf die Bemessung des Hüftgürtels erweist es sich als vorteilhaft, wenn der Hüftgürtel an vorzugsweise jeder Stelle eine Erstreckung in Längsrichtung von wenigstens 5 cm, insbesondere von wenigstens 6 cm, insbesondere von wenigstens 7 cm und insbesondere von wenigstens 8 cm aufweist. Hierbei ist die Abmessung des Hüftgürtels in Längsrichtung, also die Gürtelbreite quer zu seiner Umfangserstreckung, und zwar außerhalb des vorderen oder hinteren Latzabschnitts gemeint. Die Erstreckung des Hüftgürtels in Längsrichtung ist vorzugsweise höchstens 25 cm. Besonders bevorzugt ist die Erstreckung im Bauchbereich geringer als im Rückenbereich, und der obere Rand des Hüftgürtels ist vorzugsweise leicht konturiert, um einen Bauchausschnitt zu bilden, der die Passform des Produkts verbessert, da es sich bei Bewegung an den Träger anpasst sowie ein Rollen/Umschlagen des Gürtels im Bauchbereich verhindert.

Hinsichtlich der Bemessung des Latzabschnitts erweist es sich als vorteilhaft, wenn der Latzabschnitt eine Erstreckung in Längsrichtung über eine schrittzugewandte distale Querkante des Bauch- oder Rückenbereichs in distaler Richtung auf den Schrittbereich von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10-15 cm aufweist, wobei der vordere und hintere Latzabschnitt die gleiche oder eine unterschiedliche Erstreckung haben können. Die Erstreckung des Hüftgürtels in Längsrichtung beträgt im Bereich des Latzabschnitts vorne bei konturiertem Bauchausschnitt vorzugsweise 13 cm bis 19 cm, vorzugsweise 15 cm bis 17 cm, insbesondere 16 cm, im Bereich des Latzabschnitts hinten vorzugsweise 20 cm bis 27 cm, vorzugsweise 22 cm bis 25 cm, insbesondere 23,5 cm. Bei konturiertem Bauchausschnitt wurde dieser Wert ausgehend vom distalsten (tiefsten) Punkt der proximalen Querkante des Bauchbereichs bestimmt.

Die Erstreckung des Hüftgürtels in Querrichtung, das heisst, in Hüftumfangsrichtung beträgt zwischen 75 und 175 cm , vorzugsweise für die Größe S zwischen 50 und 90 cm, für die Größe M zwischen 80 und 120 cm, für die Größe L zwischen 100 und 140 cm, für die Größe XL zwischen 120 und 160 cm sowie für die Größe XXL zwischen 140 und 190 cm und orientiert sich an den Kleiderkonfektionsgrößen.

Die Breite der Inkontinenzvorlage an der breitesten Stelle des Produktes beträgt von 200 mm bis 400 mm, vorzugsweise 220 mm bis 320 mm, die Breite des absorbierenden Kerns beträgt von 180 mm bis 380 mm, vorzugsweise 200 mm bis 300 mm. So beträgt beispielsweise die Breite der Inkontinenzvorlage MoliForm® Premium Soft Light (Größe 1) 260 mm, die Breite des absorbierenden Kerns 242 mm, bei MoliForm® Premium Soft Extra (Größe 3) beträgt die Breite der Vorlage 300 mm, die Breite des absorbierenden Kerns 260 mm.

Nach einer bevorzugten Ausführungsform der Erfindung ist der betreffende Latzabschnitt einstückig mit dem Bauchbereich und/oder mit dem Rückenbereich des Hüftgürtels ausgebildet. Separat angestückte Latzabschnitte aus dem gleichen Material wie der übrige Hüftgürtel oder aus einem anderen Material als der übrige Hüftgürtel sind aber ebenfalls denkbar.

Der Bauchbereich und/oder Rückenbereich des Hüftgürtels ist vorzugsweise von einem Vliesmaterial oder Vliesverbundmaterial, insbesondere von einem im Wesentlichen undehnbaren Vliesmaterial oder Vliesverbundmaterial, gebildet.

Weiter erweist es sich als besonders vorteilhaft, wenn ein elastisch dehnbarer Seitenbereich des Hüftgürtels mit einem im wesentlichen undehnbaren Bauchbereich und einem im Wesentlichen undehnbaren Rückenbereich unlösbar verbunden ist. Vorzugsweise umfasst jeder Seitenbereich einen elastisch dehnbaren Abschnitt. In einer alternativen Ausführungsform kann es sich als vorteilhaft erweisen, wenn Teile des Rückenbereichs, insbesondere die seitlichen Teile des Rückenbereichs, die an die Seitenbereiche angefügt sind, elastische ausgebildet sind.

Darüber hinaus erweist es sich als vorteilhaft, wenn der vordere und/oder hintere Latzabschnitt farbig ausgebildet ist, wobei die Abschnitte vorzugsweise unterschiedlich farbig sind, um als visuelle Anlegehilfe zu dienen.

Weiterhin ist es vorteilhaft, wenn auf dem vorderen und/oder hinteren Latzabschnitt eine Markierung angebracht ist, die als Positionierhilfe für die Inkontinenzvorlage dient, derart, dass bei korrekt angelegter Inkontinenzvorlage und Fixier- und Tragevorrichtung die Markierung auf dem vorderen und/oder hinteren Latzabschnitt mit einer auf der äußeren Lage der Inkontinenzvorlage sichtbaren Markierung in Einklang gebracht ist. Beispielsweise kann eine Ausnehmung mittig am schrittzugewandten Rand des vorderen und/oder hinteren Latzabschnitts in Einklang gebracht werden mit einer Markierung, z.B. in Form eines in Längsrichtung mittig aufgebrachten Nässeindikators auf der äußeren Lage der Inkontinenzvorlage. Alternativ kann die Breite des unteren Rand des vorderen und/oder hinteren Latzabschnitts herstellerseitig so gewählt werden, dass diese mit dem Abstand von Markierungen auf der äußeren Lage der Inkontinenzvorlage, wie beispielsweise einer Größen- oder Saugstärken-Markierung, in Einklang gebracht werden kann.

Wie bereits ausgeführt, ist es besonders vorteilhaft, wenn die Fixier- und Tragevorrichtung mit auf den Hüftgürtel eingeschlagenen Verschlusselementen des Latzabschnitts im Bereich des Latzabschnitts lediglich die Längserstreckung in Längsrichtung des vorderen Bauchbereichs bzw. hinteren Rückenbereichs aufweist. Dadurch wird im eingeschlagenen Zustand über die gesamte Länge des Hüftgürtels eine gleichmäßige Erstreckung in Längsrichtung erreicht, so dass das Hindurchfädeln und -ziehen unter einer Person besonders einfach ist und es nicht zu einem Einreißen im Bereich des Übergangs von Bauch- oder Rückenbereich zu Latzabschnitt kommt.

Dies wird noch dadurch verbessert, wenn die Erstreckung des linken und rechten Seitenbereichs der Erstreckung des vorderen Bauchbereichs oder des hinteren Rückenbereichs in Längsrichtung entspricht. Der Hüftgürtel besitzt dann in der erfindungsgemäß gefalteten Konfiguration über seine gesamte Länge die gleiche Erstreckung in Längsrichtung.

Weiterhin ist es erfindungsgemäß bevorzugt, wenn der Hüftgürtel im Bereich eines der Seitenbereiche um eine in Längsrichtung verlaufende Faltlinie so auf sich selber eingeschlagen ist, dass der vordere Bauch- und der hintere Rückenbereich aufeinander zu liegen kommen. Durch diese Faltung wird die Länge des Hüftgürtels je nach Produktgröße z.B. etwa halbiert und er kann leichter gelagert und verpackt werden. Vor diesem Faltschritt sind die Gürtelverschlusselemente geöffnet und der Hüftgürtel ist in seiner vollständig ausgeklappten Konfiguration, wie er dann auch an einen Träger angelegt wird.

Zur besseren Verpackbarkeit und Entnehmbarkeit aus einer Verpackung können die nachfolgenden Schritte bzw. die nachfolgende Faltmethode ebenfalls beitragen.

Insbesondere ist vorgesehen, dass die Verschlusselemente des Latzabschnitts des vorderen Bauchbereichs und des hinteren Rückenbereichs übereinander zu liegen kommen. Hierdurch kann erreicht werden, dass bei allen Falt- und Einschlagschritten es nicht zu einer Knickung der Verschlusselemente, an denen die Inkontinenzvorlage befestigt werden kann, kommt.

Bevorzugt können die Verschlusselemente des Latzabschnitts in der eingeschlagenen Konfiguration leicht lösbar an der körperzugewandten Seite des Latzabschnitts haftend festgelegt sein. Alternativ kann aber auch nur ein Einschlagen ohne Anhaftung vorgesehen sein.

Zur weiteren Verbesserung der Verpack- und Lagerbarkeit kann der Hüftgürtel um zwei in Längsrichtung verlaufende Einschlaglinien, die beidseits des vorderen Bauch- bzw. hinteren Rückenbereichs vorgesehen sind auf sich selber einschlagbar sein. Hierdurch wird die Quererstreckung des gefalteten Produkts weiter verringert. Je nach Produktgröße erfolgt z.B.eine Reduzierung der bereits im wesentlichen halbierten Hüftumfangslänge des Hüftgürtels um in etwa die Hälfte bis zu einem Drittel.

Abschließend kann vorgesehen sein, dass das Gürtelverschlusselement im eingeschlagenen Zustand auf dem nicht das Gürtelverschlusselement tragenden Seitenteil haftend festlegbar ist. Hierdurch wird das vollständig gefaltete und zur Verpackung bereite Produkt bzw. das verpackte Produkt auf sich selber fixiert.

Dabei kann vorgesehen sein, dass beim Einschlagen um die beidseits des vorderen Bauch- und hinteren Rückenbereichs angeordneten Faltlinien zunächst der nicht die Gürtelverschlusselemente tragende Seitebereich des Hüftgürtels eingeschlagen und danach der das Gürtelverschlusselement tragende Seitebereich des Hüftgürtels eingeschlagen wird. Hierdurch wird erreicht, dass im letzten Schritt eine haftende Festlegung des Gürtelverschlusselementes auf dem Material des Hüftgürtels möglich wird, wodurch die Endfixierung möglich wird.

Bei besonders großen Hüftgürteln kann, um ein Falten auf eine geringere Größe zu ermöglichen, vorgesehen sein, dass ein Abschnitt des Seitenbereichs, der einen Teil des Gürtelverschlusselements trägt, und nach dem Einschlagschritt um die beiden beidseits des vorderen Bauchbereichs und des hinteren Rückenbereichs angeordneten Faltlinien einen überstehenden Bereich bildet, noch mal um eine in Längsrichtung verlaufende Faltlinie so eingeschlagen wird, dass das Gürtelverschlusselement auf dem Hüftgürtel festlegbar ist.

Wenn im vorstehenden der Begriff Längsrichtung verwendet wird, bezieht sich dieser stets auf die Längsachse einer den Hüftgürtel tragenden Person.

Schließlich betrifft die Erfindung noch ein Verfahren zum Falten eines Fixier- und Tragevorrichtung zur verbesserten Verpackbarkeit und Entnehmbarkeit aus einer Verpackung insbesondere mit mehreren Hüftgürteln, wobei das Verfahren die vorstehend beschriebenen Faltschritte umfasst.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Fixier- und Tragevorrichtung für eine wegwerfbare absorbierende Inkontinenzvorlage.

In der Zeichnung zeigt:
Figuren 1 und 2 eine Ausführungsform der erfindungsgemäßen Fixier- und Tragevorrichtung im angelegten Zustand zusammen mit einer wegwerfbaren absorbierenden Inkontinenzvorlage;
Figur 3 eine schematische Draufsicht auf die Fixier- und Tragevorrichtung im geöffneten und auf eine ebene Unterlage ausgebreiteten Zustand und
Figur 4 einen erfindungsgemäßen Faltvorgang.

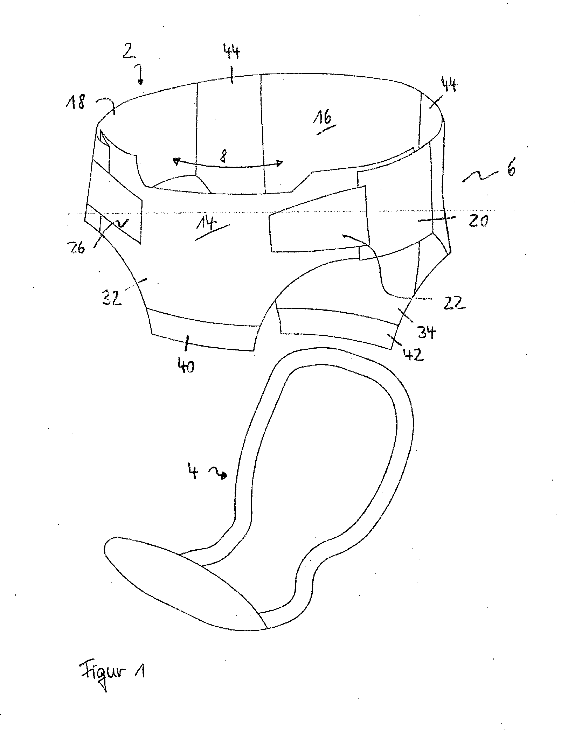

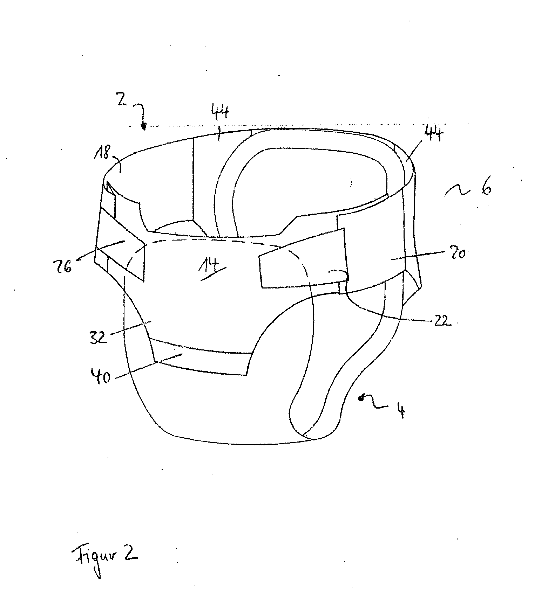

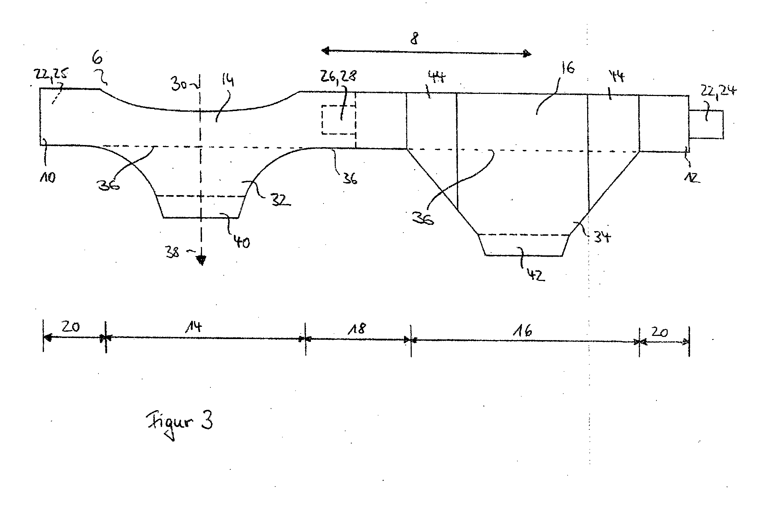

Die Figuren 1 und 2 zeigen perspektivische Ansichten einer insgesamt mit dem Bezugszeichen 2 bezeichneten Fixier- und Tragevorrichtung für eine mit Bezugszeichen 4 angedeutete absorbierende Inkontinenzvorlage, und zwar im an einen Benutzer angelegten Zustand. Die Fixier- und Tragevorrichtung 2 umfasst einen auf sich selbst lösbar schließbaren Hüftgürtel 6, der sich entlang einer Hüftumfangsrichtung 8 erstreckt und zwei freie Endabschnitte 10, 12 (s. Figur 3) aufweist, die zur Bildung der geschlossenen Hüftumfangsform auf sich selbst schließbar sind.

Die Fixier- und Tragevorrichtung 2 bzw. ihr Hüftgürtel 6 umfassen einen vorderen Bauchbereich 14, einen hinteren Rückenbereich 16 sowie einen linken Seitenbereich 18 und einen rechten Seitenbereich 20.

Zum Schließen des Hüftgürtels 6 sind an dessen freien Endabschnitten 10, 12 Gürtelverschlusselemente 22 vorgesehen, die im bevorzugt und beispielhaft dargestellten Fall von mechanisch wirkenden Verschlusselementen in Form eines Haken/Schlaufenverschlusssystems gebildet sind. Dabei ist in den Figuren mit Bezugszeichen 24 die hakenförmige Verschlusskomponente dargestellt, die über den Seitenrand 12 des Hüftgürtels hinausragt und mit einer schlaufenbildenden Komponente 25 in Form der äußeren Oberfläche des Bauchbereichs 14 lösbar haftend zusammenwirkt. Beispielsweise weist die Außenseite des Bauchbereichs 14 ein Vliesmaterial auf, welches die schlaufenförmige Komponente 25 des Verschlusssystems bildet.

Des Weiteren weist der nur an seinen Endabschnitten 10, 12, also nur an einer Stelle öffenbare Hüftgürtel 6 Sekundärverschlusselemente 26 auf, mittels derer die Umfangslänge des Hüftgürtels 6 einstellbar ist. Die Sekundärverschlusselemente 26 sind seitlich am Bauchbereich 14 bzw. am linken Seitenbereich 18 so vorgesehen, dass sie im angelegten Zustand der Fixier- und Tragevorrichtung (s. Figur 1) denselben optischen Eindruck wie die Gürtelverschlusselemente 22 hinsichtlich Ausbildung und Anordnung vermitteln, obschon ihre Funktion verschieden ist. Mittels der Sekundärverschlusselemente 26 werden nämlich keine öffenbaren und voneinander trennbaren Endabschnitte des Hüftgürtels 6 miteinander verbunden, sondern die Sekundärverschlusselemente 26 dienen lediglich dazu, den bestimmungsgemäßen Sitz des Hüftgürtels 6 an die Tragesituation anzupassen. Hierfür umfassen die Sekundärverschlusselemente 26 einen auf die Außenseite des Hüftgürtels 6 aufgebrachten Materialabschnitt 28, auf dem oder an dem sie selbst vorgesehen sind. Bei den Sekundärverschlusselementen 26 handelt es sich wiederum in bevorzugter Weise um mechanisch wirkende Verschlusselemente, insbesondere um Haken/Schlaufenmaterialien. Im beispielhaft dargestellten und bevorzugten Fall sind die Sekundärverschlusselemente 26 derart ausgebildet, dass an dem Materialabschnitt 28 eine hakenbildende Komponente des Verschlusssystems vorgesehen ist, die mit einer schlaufenbildenden Außenseite des Bauchbereichs 14 zusammenwirkt, so wie dies vorausgehend bei den Gürtelverschlusselementen 22 beschrieben wurde.

Als weitere wesentliche Komponente umfasst der Hüftgürtel 6 ausgehend vom Bauchbereich 14 und vom Rückenbereich 16 je einen symmetrisch zu einer Längsmittelachse 30 ausgebildeten vorderen und hinteren Latzabschnitt 32, 34. Die Latzabschnitte 32, 34 sind im beispielhaft bevorzugt dargestellten Fall einstückig mit dem Hüftgürtel 6 ausgebildet; sie erstrecken sich von einer schrittzugewandten oder unteren distalen Querkante 36 des Hüftgürtels entlang der Längsmittelachse 30 in Richtung 38 auf den Schrittbereich. Sie können sich in Richtung auf den Schrittbereich verjüngen und seitlich bogenförmig konturiert ausgebildet sein. An ihrem unteren, schrittzugewandten Endbereich umfassen die Latzabschnitte 32, 34 mechanisch wirkende Verschlusselemente 40, 42, und zwar auf der körperzugewandten Seite. Diese mechanisch wirkenden Verschlusselemente 40, 42 wirken lösbar haftend mit komplementären mechanisch wirkenden Verschlusselementen auf der körperabgewandten Seite der Inkontinenzvorlage 4 zusammen, um die Inkontinenzvorlage 4 lösbar, jedoch verliersicher an der Fixier- und Tragevorrichtung 2 festzulegen. Bevorzugtermaßen handelt es sich bei den Verschlusselementen 40, 42 um die hakenbildende Komponente eines mechanisch wirkenden Verschlusssystems, die mit der schlaufenbildenden Komponente auf der körperabgewandten Seite der Inkontinenzvorlage 4 zusammenwirkt. Die schlaufenbildende Komponente kann dabei in vorteilhafter Weise von einer Vliesbeschichtung, vorzugsweise der Vlieslage eines Vlies-Folien-Laminates, der Inkontinenzvorlage 4 gebildet sein.

Der Hüftgürtel 6 ist vorzugsweise aus Vlies- oder Vliesverbundmaterialien hergestellt. Im beispielhaft dargestellten jedoch bevorzugten Fall sind der Bauchbereich 14 und die beiden Seitenbereiche 18, 20 aus einem im Wesentlichen undehnbaren Vliesmaterial oder Vliesverbundmaterial gebildet, dessen Außenseite als schlaufenbildende Komponente für die Gürtelverschlusselemente 22 bzw. die Sekundärverschlusselemente 26 dient. Der Rückenbereich 16 ist zumindest teilweise elastisch dehnbar ausgebildet, indem er ein Paar elastisch dehnbare Materialabschnitte 44 aufweist, die bestimmungsgemäß unlösbar mit dem undehnbaren Material der Seitenbereiche 18, 20 verbunden sind und zwischen sich einen elastisch undehnbaren Bereich des Rückenbereichs einschließen.

Das Anlegen der Fixier- und Tragevorrichtung 2 zusammen mit der absorbierenden Inkontinenzvorlage 4 geschieht folgendermaßen: Zunächst wird der Hüftgürtel 6 bei bettlägerigen pflegebedürftigen Patienten durch eine Pflegeperson unter dem Körper des Patienten auf Hüfthöhe hindurchgeführt. Der Hüftgürtel 6 wird dabei so positioniert, dass der Rückenbereich 16 unterhalb des Gesäßes und der Bauchbereich 14 ungefähr vorne mittig zu liegen kommen. Sodann werden die freien Endabschnitte 10, 12 des Hüftgürtels 6 übereinander positioniert und mittels der Gürtelverschlusselemente 22 aufeinander geschlossen. Durch anschließendes Positionieren und Schließen des Materialabschnitts 28 und der Sekundärverschlusselemente 26 wird die Länge und Spannung des Hüftgürtels 6 in Umfangsrichtung 8 optimiert, so dass ein gleichmäßiger Zug auf den Bauchbereich 14 links und rechts ausgeübt wird und der Bauchbereich 14 wie auch der Rückenbereich 16 vorzugsweise symmetrisch in Bezug auf den Schrittbereich des Patienten und möglichst faltenfrei zu liegen kommen. Hierbei ist auch auf ein angenehmes Traggefühl abzustellen. Sodann wird der vordere und hintere Latzabschnitt 32, 34 mit der zuvor oder erst jetzt im Schrittbereich des Patienten positionierten Inkontinenzvorlage 4 unter Verwendung der mechanisch wirkenden Verschlusselemente 40, 42 lösbar haftend verbunden. Auch hierbei ist darauf zu achten, dass die vorderen und hinteren Latzabschnitte 32, 34 unter Ausübung einer gleichmäßigen und moderaten Zug- bzw. Haltekraft auf die Inkontinenzvorlage 4 fixiert werden, damit die Inkontinenzvorlage 4 in ihrer bestimmungsgemäßen symmetrischen Anordnung im Schrittbereich des Patienten verbleibt.

Hinsichtlich der Reihenfolge der vorausgehend geschilderten Schritte zum Anlegen des Hygieneartikels bzw. Inkontinenzsystems ist die Pflegeperson verhältnismäßig frei. Beispielsweise kann es sich in bestimmten Pflegesituationen als vorteilhaft erweisen, dass zunächst die Inkontinenzvorlage im Schrittbereich des Patienten vorpositioniert wird und erst dann der Hüftgürtel 6 auf sich selbst geschlossen wird. Es kann sich auch als zweckmäßig erweisen, dass die Sekundärverschlusselemente 26 erst nach dem Verbinden der Latzabschnitte 32, 34 mit der Inkontinenzvorlage 4 genutzt werden, um die optimale Spannung in Hüftumfangsrichtung 8 vorzugeben.

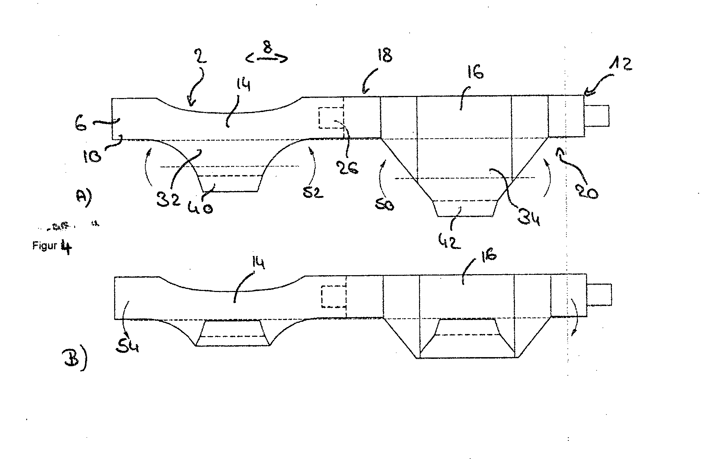

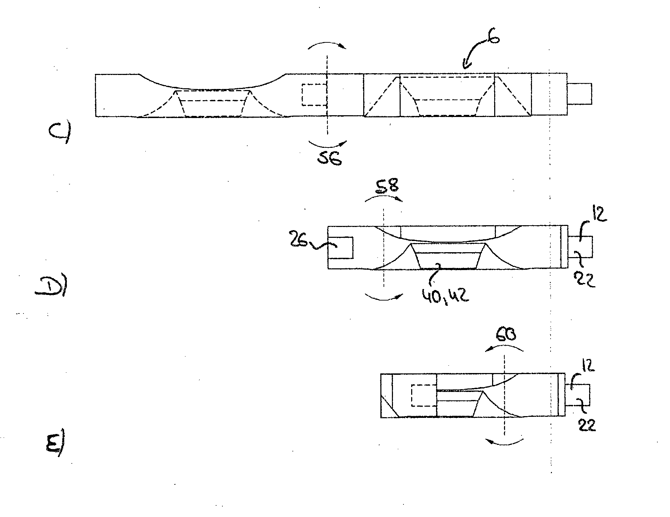

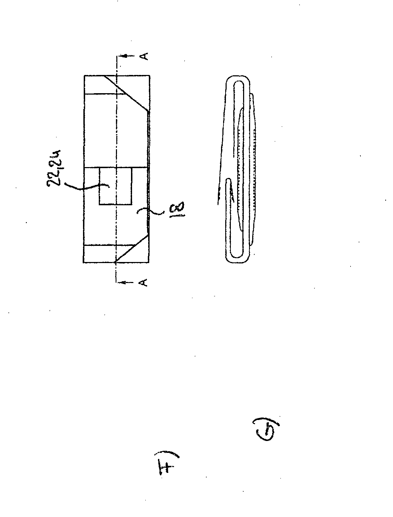

Figur 4 zeigt nun, wie ein erfindungsgemäßer Gegenstand zur vereinfachten Verpackbarkeit und Entnehmbarkeit aus einer Verpackung, insbesondere auch wenn mehrere Fixier- und Tragevorrichtungen 2 in einer Verpackung angeordnet sind, gefaltet und zusammengelegt werden kann. Die Fixier- und Tragevorrichtung 2 ist hierbei in Darstellung A) in Draufsicht auf die Innenseite mit seinen zwei freien Endabschnitten in einem aufgeklappten Zustand gezeigt, die zur Bildung der geschlossenen Hüftumfangsform auf sich selbst schließbar sind. Im vorderen Bauchbereich 14 sowie im hinteren Rückenbereich 16 ist an den Bauch-14 und Rückenbereich 16 jeweils ein Latzabschnitt 32 und 34 angeschlossen, an dessen unterem, einem Schrittbereich zugewandten Randbereich ein Verschlusselement 40 bzw. 42 angeordnet ist. Dabei wird nun der untere Teil der Latzabschnitte 32 und 34, die die sich vorzugsweise über die gesamte Breite der Latzabschnitte erstreckenden, vorzugsweise einstückigen Verschlusselemente 40 und 42 tragen, um eine Faltlinie 50, 52 nach oben auf den oberen Bereich der Latzabschnitte 32 und 34 umgeschlagen, so dass die Verschlusselemente 40 und 42 auf dem Material der Latzabschnitte haftend zu liegen kommen, indem die Verschlusselemente 40 und 42, die im Tragezustand auf einen Träger zu gerichtet sind, mit dem auf einen Benutzer weisenden Material des Latzabschnitts 32 bzw. 34 haftend, insbesondere mechanisch haftend, zusammenwirken. In einem nächsten Faltschritt, der in der Darstellung B) gezeigt ist, erfolgt dann ein Einschlagen des oberen Latzbereiches der Latzabschnitte 32 und 34 um eine mit 54 bezeichnete Einschlaglinie, so dass die Latzbereiche mit darauf eingeschlagenen Verschlusselementen auf dem hinteren Rückenbereich 16 bzw. dem vorderen Bauchbereich 14 zu liegen kommen. Nach diesem Falt- und Einschlagschritt weist der Hüftgürtel 6 im Wesentlichen über seine gesamte Länge die gleiche Erstreckung in Längsrichtung, die in Darstellung C) mit dem Bezugszeichen L gekennzeichnet ist, auf.

Ein entsprechend eingeschlagener Hüftgürtel 6 ist in Darstellung C) gezeigt. Der Hüftgürtel 6 wird dann um eine in Längsrichtung verlaufende Einschlaglinie 56, die somit im rechten Winkel zu den Einschlaglinien 50 bis 54 verläuft, so auf sich selber eingeschlagen, dass die Verschlusselemente 40 und 42 im eingeschlagenen Zustand übereinander zu liegen kommen. Der Hüftgürtel 6 wird dabei im Wesentlichen hinsichtlich seiner Längserstreckung halbiert.

Nach diesem Faltschritt ragt der freie Endabschnitt 12, der das Gürtelverschlusselement 22 mit der hakenbildenden Komponente 24 trägt, über den freien Endabschnitt 10 in Querrichtung hinaus. Die Faltlinie wird dabei so gewählt, dass ein Sekundärverschlusselement 26 nicht durch die Faltlinie geknickt wird. Es erfolgt nun ein in Darstellung D) gezeigter weiterer Faltschritt um eine in Längsrichtung verlaufende Faltlinie 58, wobei die Faltlinie 58 nicht mit den Verschlusselementen 40 und 42 in Überlappung gerät derart, dass der Seitenteil 18, der das Sekundärverschlusselement 26 aufweist, um diese Linie so eingeschlagen wird, dass das Sekundärverschlusselement 26 im Bereich des Rücken- und Bauchbereichs 16,14 zu liegen kommt. In einem nachfolgenden Faltschritt, der in Darstellung E) gezeigt ist, wird um eine weitere in Längsrichtung verlaufende Faltlinie 60 der zweite Seitenteil 20 ebenfalls eingeschlagen, wobei dann der freie Endabschnitt 12 mit dem Gürtelverschlusselement 22 so zu liegen kommt, dass er mit seinen insbesondere mechanischen und/oder klebenden Verschlusselementen, insbesondere mit der hakenbildenden Komponente 24, auf dem Außenmaterial des Seitenteils 18 haftend festgelegt werden kann. Die endgültige Faltung lässt sich dabei der Darstellung F) entnehmen.

Darstellung G) zeigt den gefalteten Hüftgürtel gemäß Darstellung F) in einer Schnittdarstellung entlang der Linie A-A.

Sofern es sich um eine Fixier- oder Tragevorrichtung mit großer Hüftumfangsweite handelt, kann des Weiteren vorgesehen sein, dass der Seitenteil 20, insbesondere der freie Endabschnitt 12, der die hakenbildende Komponente 24 des Gürtelverschlusselementes 22 trägt, einmal um die fertig gefaltete Fixier- und Tragevorrichtung 2 herumgeschlungen wird und erst dann eine Festlegung der hakenbildenden Komponente 24 desGürtelverschlusselements 22 auf dem Außenmaterial des Hüftgürtels 6 erfolgt.

Auf diese Weise kann eine möglichst einfache Fixierung sämtlicher Verschlusselemente 22, 26, 40, 42 im verpackten Zustand erfolgen, so dass verhindert werden kann, dass beim Verpacken bzw. Entnehmen aus einer Verpackung die Verschlusselemente, d. h. sowohl die Sekundär-, die Gürtelverschlusselemente als auch die Verschlusselemente zum Festlegen der Inkontinenzvorlage, mit einer weiteren Fixier- und Tragevorrichtung haftend zusammenwirken und so eine einfache Entnehmbarkeit verhindern.

Darüber hinaus wird auch erreicht, dass beim Anlegen der Fixier- und Tragevorrichtung an einen Patienten durch das Einschlagen der Latzabschnitte es zu keinem versehentlichen Anhaften der Verschlusselemente 40 und 42 an Bekleidungsstücken oder anderen Materialien kommt.

## Patentansprüche

1. Fixer- und Tragevorrichtung (2) für eine wegwerfbare absorbierende Inkontinenzvorlage (4) mit einem mittels Gürtelverschlusselementen (22) auf sich selbst lösbar schließbaren und so eine in Hüftumfangsrichtung (8) durchgehende Hüftöffnung bildenden Hüftgürtel (6), an den die Inkontinenzvorlage (4) lösbar anbringbar ist, so dass sie im Schrittbereich des Benutzers getragen und nach Gebrauch wieder vom Hüftgürtel (6) entfernt und verworfen werden kann, wobei der Hüftgürtel (6) einen vorderen Bauchbereich (14), einen hinteren Rückenbereich (16) und einen linken und rechten Seitenbereich (18, 20) umfasst, der Hüftgürtel (6) ausgehend von dem Rückenbereich (16) und dem Bauchbereich (14) je einen sich in einer Längsrichtung in Richtung (38) auf den Schrittbereich des Benutzers erstreckenden Latzabschnitt (32, 34) aufweist, der an seiner körperzugewandten Seite Verschlusselemente (40, 42) aufweist, die mit komplementären Verschlusselementen auf der körperabgewandten Seite der Inkontinenzvorlage (4) lösbar haftend zusammenwirken, wobei die Verschlusselemente (40, 42) der Latzabschnitte (32, 34) vor der Ingebrauchnahme auf die körperzugewandte Seite des Latzabschnitts (32, 34) eingeschlagen sind, **dadurch gekennzeichnet, dass** die Latzabschnitte (32, 34) mit den darauf eingeschlagenen Verschlusselementen (40, 42) des Latzabschnitts (32, 34) auf den vorderen Bauch-(14) und hinteren Rückenbereich (16) des Hüftgürtels eingeschlagen sind.

2. Fixier- und Tragevorrichtung nach Anspruch 1, **dadurch gekennzeichnet, dass** die Fixier- und Tragevorrichtung (2) mit auf den Bauch- und Rückenbereich (14,16) eingeschlagenen Verschlusselementen (40, 42) des Latzabschnitts (32, 34) im Bauch- und Rückenbereich (14,16) lediglich die Erstreckung in Längsrichtung des vorderen Bauchbereichs (14) oder hinteren Rückenbereichs (16) aufweist.

3. Fixier- und Tragevorrichtung nach Anspruch 1 oder 2, **dadurch gekennzeichnet, dass** die Erstreckung des linken und rechten Seitenbereichs (18, 20) in Längsrichtung der Erstreckung des vorderen Bauchbereichs (14) und/oder des hinteren Rückenbereichs (16) in Längsrichtung entspricht.

4. Fixier- und Tragevorrichtung nach einem oder mehreren der vorangehenden Ansprüche, **dadurch gekennzeichnet, dass** der Hüftgürtel (6) im Bereich eines der Seitenbereiche (18, 20) um eine in Längsrichtung verlaufende Faltlinie (56) so auf sich selber eingeschlagen ist, dass der vordere Bauch-(14) und der hintere Rückenbereich (16) aufeinander zu liegen kommen.

5. Fixier- und Tragevorrichtung nach Anspruch 4, **dadurch gekennzeichnet, dass** die Verschlusselemente (40, 42) des Latzabschnitts (32, 34) des vorderen Bauchbereichs (14) und des hinteren Rückenbereichs (16) übereinander zu liegen kommen.

6. Fixier- und Tragevorrichtung nach Anspruch 4 oder 5, **dadurch gekennzeichnet, dass** der Hüftgürtel (6) um zwei in Längsrichtung verlaufende Faltlinien (58, 60), die beidseits des vorderen Bauch- (14) bzw. hinteren Rückenbereichs (16) und außerhalb der Verschlusselemente (40, 42) der Latzabschnitte (32, 34) vorgesehen sind, auf sich selber einschlagbar ist.

7. Fixier- und Tragevorrichtung nach Anspruch 6, **dadurch gekennzeichnet, dass** beim Einschlagen um die beidseits des vorderen Bauch- (14) und hinteren Rückenbereichs (16) angeordneten Faltlinien (58, 60) zunächst der nicht die Gürtelverschlusselemente (22) tragende Seitenbereich (18) des Hüftgürtels (6) eingeschlagen und danach der die Gürtelverschlusselemente (22) tragende Seitebereich des Hüftgürtels (6) eingeschlagen wird.

8. Fixier- und Tragevorrichtung nach Anspruch 7, **dadurch gekennzeichnet, dass** ein Gürtelverschlusselement (22) im eingeschlagenen Zustand auf dem nicht Gürtelverschlusselemente (22) tragenden Seitenteil (18) haftend festlegbar ist.

9. Fixier- und Tragevorrichtung nach Anspruch 7, **dadurch gekennzeichnet, dass** ein Abschnitt des Seitenbereichs (20) mit dem freien Endabschnitt 12, der einen Teil (24) des Gürtelverschlusselements (22) trägt, und nach dem Einschlagschritt um die beiden beidseits des vorderen Bauchbereichs (14) und des hinteren Rückenbereichs (16) angeordneten Faltlinien (58, 60), noch mal um eine in Längsrichtung verlaufende Faltlinie so um den gefalteten Hüftgürtel (6) eingeschlagen wird, dass das Gürtelverschlusselement (22, 24) auf dem Hüftgürtel (6) festlegbar ist.

10. Fixier- und Tragevorrichtung nach einem oder mehreren der vorangehenden Ansprüche, **dadurch gekennzeichnet, dass** die Verschlusselemente (40, 42) des Latzabschnitts (32, 34) in der eingeschlagenen Konfiguration leicht lösbar an der körperzugewandten Seite des Latzabschnitts (32, 34) haftend festgelegt sind.

11. Fixier- und Tragevorrichtung nach einem oder mehreren der vorangehenden Ansprüche, **dadurch gekennzeichnet, dass** die auf den vorderen Bauch- und hinteren Rückenbereich (14,16) eingeschlagenen Latzabschnitte (32,34) dort lösbar, insbesondere mittels Ultraschallschweiß- oder Klebeverbindungen fixiert sind.

12. Fixier- und Tragevorrichtung nach einem oder mehreren der vorangehenden Ansprüche, **dadurch gekennzeichnet, dass** Sekundärverschlusselemente (26) vorgesehen sind, mittels derer eine Umfangslänge des Hüftgürtels (6) einstellbar und so zumindest der vordere Latzabschnitt (32) symmetrisch zum Schritt des Benutzers positionierbar ist, wobei die Sekundärverschlusselemente (26) im Seitenbereich (18) des Hüftgürtels (6), gegenüberliegend den Gürtelverschlusselementen (22) vorgesehen sind.

13. Fixier- und Tragevorrichtung nach einem oder mehreren der vorangehenden Ansprüche, **dadurch gekennzeichnet, dass** die Gürtelverschlusselemente (22) und/oder die Sekundärverschlusselemente (26) und/oder die Verschlusselemente (40,42) der Latzabschnitte (32,34) mechanische und/oder klebende Verschlusselemente sind.

14. Fixier- und Tragevorrichtung nach einem oder mehreren der vorangehenden Ansprüche, **dadurch gekennzeichnet, dass** der Hüftgürtel (6) nur in einem Bereich öffen- und schließbar und die Gürtelverschlusselemente (22) in diesem Bereich an freien Endabschnitten (10,12) des Hüftgürtels (6) vorgesehen sind, wobei die Gürtelverschlusselemente (22) in einem oder beiden Seitenbereichen (18,20) des Hüftgürtels (6) vorgesehen sind.

15. Fixier- und Tragevorrichtung nach einem oder mehreren der vorangehenden Ansprüche, **dadurch gekennzeichnet, dass** der Hüftgürtel (6) nur in einem Bereich öffen- und schließbar und die Gürtelverschlusselemente (22,24,25) in diesem Bereich an einem freien Endabschnitt (10,12) des Hüftgürtels (6) vorgesehen sind.

16. Fixier- und Tragevorrichtung nach einem oder mehreren der vorangehenden Ansprüche, **dadurch gekennzeichnet, dass** die Verschlusselemente (40,42) des Latzabschnitts auf einem streifenförmigen und quer zur Längsrichtung erstreckten Materialabschnitt vorgesehen sind, der an den Latzabschnitt angefügt ist.

## Claims

1. A fixing and carrying device (2) for a disposable absorbent incontinence pad (4), which is detachably closable on itself by means of girdle closure elements (22) and thus forms a girdle (6) with a continuous hip opening in the hip circumferential direction (8), the incontinence pad (4) being detachably attachable to said girdle (6) such that it can be worn in the crotch region of the user and can be removed from the girdle (6) again and discarded after use, wherein the girdle (6) comprises a front stomach region (14), a rear back region (16) and a left-hand and a right-hand side region (18, 20), the girdle (6), starting from the back region (16) and the stomach region (14), has in each case one flap section (32, 34) extending in a longitudinal direction in the direction (38) of the crotch region of the user, having, on its side facing the body, closure elements (40, 42) which interact in a detachably adhesive manner with complementary closure elements on that side of the incontinence pad (4) that faces away from the body, wherein the closure elements (40, 42) of the flap sections (32, 34), prior to being put into use, are folded in onto that side of the flap section (32, 34) that faces the body, **characterized in that** the flap sections (32, 34), onto which the closure elements (40, 42) of the flap section (32, 34) are folded in, are folded in onto the front stomach region (14) and rear back region (16) of the girdle.

2. The fixing and carrying device as claimed in claim 1, **characterized in that** the fixing and carrying device (2) having closure elements (40, 42), folded in onto the stomach and back region (14, 16), of the flap section (32, 34) has, in the stomach and back region (14, 16), merely the extent in the longitudinal direction of the front stomach region (14) or rear back region (16).

3. The fixing and carrying device as claimed in claim 1 or 2, **characterized in that** the extent of the left-hand and right-hand side region (18, 20) in the longitudinal direction corresponds to the extent of the front stomach region (14) and/or of the rear back region (16) in the longitudinal direction.

4. The fixing and carrying device as claimed in one or more of the preceding claims, **characterized in that** the girdle (6) is folded in on itself in the region of one of the side regions (18, 20) about a folding line (56) extending in the longitudinal direction, such that the front stomach region (14) and the rear back region (16) come to lie on one another.

5. The fixing and carrying device as claimed in claim 4, **characterized in that** the closure elements (40, 42) of the flap section (32, 34) of the front stomach region (14) and of the rear back region (16) come to lie over one another.

6. The fixing and carrying device as claimed in claim 4 or 5, **characterized in that** the girdle (6) is folded in on itself about two folding lines (58, 60) extending in the longitudinal direction, being provided on both sides of the front stomach region (14) and rear back region (16) and outside the closure elements (40, 42) of the flap sections (32, 34).

7. The fixing and carrying device as claimed in claim 6, **characterized in that**, in the case of folding in about the folding lines (58, 60) arranged on both sides of the front stomach region (14) and rear back region (16), first of all that side region (18) of the girdle (6) that does not bear the girdle closure elements (22) is folded in and then that side region of the girdle (6) that bears the girdle closure elements (22) is folded in.

8. The fixing and carrying device as claimed in claim 7, **characterized in that** a girdle closure element (22) is securable in an adhesive manner in the folded-in state to the side part (18) not bearing girdle closure elements (22).

9. The fixing and carrying device as claimed in claim 7, **characterized in that** a section of the side region (20) having the free end section (12) which bears a part (24) of the girdle closure element (22), and after the folding-in step about the two folding lines (58, 60) arranged on both sides of the front stomach region (14) and the rear back region (16), is folded in once again about a folding line extending in the longitudinal direction of the folded girdle (6), such that the girdle closure element (22, 24) is securable to the girdle (6).

10. The fixing and carrying device as claimed in one or more of the preceding claims, **characterized in that** the closure elements (40, 42) of the flap section (32, 34) are adhesively secured in the folded-in configuration in an easily detachable manner to that side of the flap section (32, 34) that faces the body.

11. The fixing and carrying device as claimed in one or more of the preceding claims, **characterized in that** the flap sections (32, 34) folded in onto the front stomach and rear back region (14, 16) are fixed in a detachable manner there, in particular by means of ultrasonic welded connections or adhesive connections.

12. The fixing and carrying device as claimed in one or more of the preceding claims, **characterized in that** secondary closure elements (26) are provided, by means of which a circumferential length of the girdle (6) is settable and thus at least the front flap section (32) is positionable symmetrically with respect to the crotch of the user, wherein the secondary closure elements (26) are provided in the side region (18) of the girdle (6), opposite the girdle closure elements (22).

13. The fixing and carrying device as claimed in one or more of the preceding claims, **characterized in that** the girdle closure elements (22) and/or the secondary closure elements (26) and/or the closure elements (40, 42) of the flap sections (32, 34) are mechanical and/or adhesive closure elements.

14. The fixing and carrying device as claimed in one or more of the preceding claims, **characterized in that** the girdle (6) is openable and closable only in one region and the girdle closure elements (22) are provided in this region on free end sections (10, 12) of the girdle (6), wherein the girdle closure elements (22) are provided in one or both side regions (18, 20) of the girdle (6).

15. The fixing and carrying device as claimed in one or more of the preceding claims, **characterized in that** the girdle (6) is openable and closable only in one region and the girdle closure elements (22, 24, 25) are provided in this region on a free end section (10, 12) of the girdle (6).

16. The fixing and carrying device as claimed in one or more of the preceding claims, **characterized in that** the closure elements (40, 42) of the flap section are provided on a strip-like material section extending transversely to the longitudinal direction, said material section being joined to the flap section.

## Revendications

1. Dispositif de fixation et de support (2) pour une protection d'incontinence (4) absorbante jetable, comprenant une ceinture de hanches (6) qui peut être fermée sur elle-même de manière amovible au moyen d'éléments de fermeture de ceinture (22) et forme ainsi une ouverture de hanches traversante dans la direction circonférentielle des hanches (8) et sur laquelle la protection d'incontinence (4) peut être fixée de manière amovible de sorte qu'elle peut être portée dans la zone d'entrejambe de l'utilisateur et peut, après l'usage, être détachée à nouveau de la ceinture de hanches (6) et être jetée, ladite ceinture de hanches (6) comprenant une zone ventrale avant (14), une zone dorsale arrière (16) et des zones latérales gauche et droite (18, 20), la ceinture de hanches (6) présentant, à partir de ladite zone dorsale (16) et de ladite zone ventrale (14), respectivement une portion-bavette (32, 34) qui s'étend dans une direction longitudinale vers (38) ladite zone d'entrejambe de l'utilisateur et qui présente, sur sa face tournée vers le corps, des éléments de fermeture (40, 42) qui coopèrent par adhérence amovible avec des éléments de fermeture complémentaires présents sur la face de la protection d'incontinence (4), qui est opposée au corps, les éléments de fermeture (40, 42) des portions-bavettes (32, 34) étant rabattus, avant la mise en usage, sur la face de la portion-bavette (32, 34) qui est tournée vers le corps, **caractérisé par le fait que** lesdites portions-bavettes (32, 34) avec les éléments de fermeture (40, 42) de la portion-bavette (32, 34) rabattus sur celles-ci sont rabattues sur les zone ventrale avant (14) et dorsale arrière (16) de la ceinture de hanches.

2. Dispositif de fixation et de support selon la revendication 1, **caractérisé par le fait que** le dispositif de fixation et de support (2) avec les éléments de fermeture (40, 42) de la portion-bavette (32, 34) dans les zones ventrale et dorsale (14, 16), qui sont rabattus sur les zones ventrale et dorsale (14,16), présente uniquement l'extension dans la direction longitudinale de la zone ventrale avant (14) ou de la zone dorsale arrière (16).

3. Dispositif de fixation et de support selon la revendication 1 ou 2, **caractérisé par le fait que** l'extension des zones latérales gauche et droite (18, 20) dans la direction longitudinale correspond à l'extension de la zone ventrale avant (14) et/ou de la zone dorsale arrière (16) dans la direction longitudinale.

4. Dispositif de fixation et de support selon l'une ou plusieurs des revendications précédentes, **caractérisé par le fait que** la ceinture de hanches (6) est rabattue sur elle-même au niveau de l'une des zones latérales (18, 20), suivant une ligne de pliage (56) s'étendant dans la direction longitudinale, de telle manière que les zones ventrale avant (14) et dorsale arrière (16) viennent se positionner l'une sur l'autre.

5. Dispositif de fixation et de support selon la revendication 4, **caractérisé par le fait que** les éléments de fermeture (40, 42) de la portion-bavette (32, 34) de la zone ventrale avant (14) et de la zone dorsale arrière (16) viennent se positionner les uns sur les autres.

6. Dispositif de fixation et de support selon la revendication 4 ou 5, **caractérisé par le fait que** ladite ceinture de hanches (6) peut être rabattue sur elle-même suivant deux lignes de pliage (58, 60) qui s'étendent dans la direction longitudinale et qui sont prévues de part et d'autre de la zone ventrale avant (14) ou bien de la zone dorsale arrière (16) et à l'extérieur des éléments de fermeture (40, 42) des portions-bavettes (32, 34).

7. Dispositif de fixation et de support selon la revendication 6, **caractérisé par le fait que**, lors du rabattement suivant les lignes de pliage (58, 60) disposées de part et d'autre des zones ventrale avant (14) et dorsale arrière (16), c'est d'abord la zone latérale (18) de la ceinture de hanches (6) ne portant pas les éléments de fermeture de ceinture (22) qui est rabattue et c'est ensuite la zone latérale de la ceinture de hanches (6) portant les éléments de fermeture de ceinture (22) qui est rabattue.

8. Dispositif de fixation et de support selon la revendication 7, **caractérisé par le fait que**, en état rabattu, un élément de fermeture de ceinture (22) peut être fixé par adhérence sur la partie latérale (18) ne portant pas d'éléments de fermeture de ceinture (22).

9. Dispositif de fixation et de support selon la revendication 7, **caractérisé par le fait qu'**une portion de la zone latérale (20) avec la portion terminale libre (12) qui porte une partie (24) de l'élément de fermeture de ceinture (22), et après l'étape de rabattement suivant les deux lignes de pliage (58, 60) disposées de part et d'autre de la zone ventrale avant (14) et de la zone dorsale arrière (16), est rabattue une nouvelle fois suivant une ligne de pliage s'étendant dans la direction longitudinale, sur la ceinture de hanches (6) pliée, de telle sorte que l'élément de fermeture de ceinture (22, 24) peut être fixé sur la ceinture de hanches (6).

10. Dispositif de fixation et de support selon l'une ou plusieurs des revendications précédentes, **caractérisé par le fait que**, en configuration rabattue, les éléments de fermeture (40, 42) de la portion-bavette (32, 34) sont fixés par adhérence, de manière à être facilement amovible, sur la face de la portion-bavette (32, 34) qui montre vers le corps.

11. Dispositif de fixation et de support selon l'une ou plusieurs des revendications précédentes, **caractérisé par le fait que** les portions-bavettes (32, 34) rabattues sur les zones ventrale avant et dorsale arrière (14, 16) y sont fixées de manière amovible, en particulier au moyen de liaisons par soudage par ultrasons ou collées.

12. Dispositif de fixation et de support selon l'une ou plusieurs des revendications précédentes, **caractérisé par le fait que** des éléments de fermeture secondaires (26) sont prévus par le bais desquels on peut régler une longueur circonférentielle de ladite ceinture de hanches (6) et, ainsi, positionner au moins la portion-bavette (32) avant de manière symétrique par rapport à l'entrejambe de l'utilisateur, lesdits éléments de fermeture secondaires (26) étant prévus dans la zone latérale (18) de la ceinture de hanches (6), en regard des éléments de fermeture de ceinture (22).

13. Dispositif de fixation et de support selon l'une ou plusieurs des revendications précédentes, **caractérisé par le fait que** les éléments de fermeture de ceinture (22) et/ou les éléments de fermeture secondaires (26) et/ou les éléments de fermeture (40, 42) des portions-bavettes (32, 34) sont des éléments de fermeture mécaniques et/ou collants.

14. Dispositif de fixation et de support selon l'une ou plusieurs des revendications précédentes, **caractérisé par le fait que** la ceinture de hanches (6) ne peut être fermée et ouverte que dans une zone et que les éléments de fermeture de ceinture (22) sont prévus dans cette zone sur des portions terminales libres (10, 12) de la ceinture de hanches (6), les éléments de fermeture de ceinture (22) étant prévus dans une ou les deux zones latérales (18, 20) de la ceinture de hanches (6).

15. Dispositif de fixation et de support selon l'une ou plusieurs des revendications précédentes, **caractérisé par le fait que** la ceinture de hanches (6) ne peut être fermée et ouverte que dans une zone et que les éléments de fermeture de ceinture (22, 24, 25) sont prévus dans cette zone sur une portion terminale libre (10, 12) de la ceinture de hanches (6).

16. Dispositif de fixation et de support selon l'une ou plusieurs des revendications précédentes, **caractérisé par le fait que** les éléments de fermeture (40, 42) de la portion-bavette sont prévus sur une portion de matière en forme de bande et s'étendant transversalement à la direction longitudinale qui est rapportée à la portion-bavette.
